# FASCICULE DE BREVET EUROPEEN

(11) **EP 0 671 896 B1**
(45) Date de publication et mention de la délivrance du brevet: **22.10.1997**
(21) Numéro de dépôt: 94901984.8
(22) Date de dépôt: 03.12.1993
(51) Int. Cl.: A61F 2/30, A61L 27/00

(54) **DISPOSITIF DE POSITIONNEMENT POUR PROTHESE TOTALE DE HANCHE**
POSITIONIEREINRICHTUNG FÜR TOTALE HÜFTPROTHESE
DEVICE FOR POSITIONING A TOTAL HIP PROSTHESIS

(30) Priorité: 03.12.1992 FR 9214787
(43) Date de publication de la demande: 20.09.1995
(73) Titulaire: PROTEK SYNTHES S.A., 25461 Etupes Cédex (FR); Aebi, Jürg, 12800 Onet-le-Château (FR); Weisang, Claude, 76550 Offranville (FR); Chaix, Claude, 13008 Marseille (FR)
(72) Inventeur: AEBI, Jürg, F-12800 Onet-le-Château (FR); WEISANG, Claude, F-76550 Offranville (FR); CHAIX, Claude, F-13008 Marseille (FR); BOURALY, Jean-Pierre, F-25200 Montbéliard (FR)
(74) Mandataire: Schmit, Christian Norbert Marie
(86) Numéro de dépôt international: FR9301192
(87) Numéro de publication internationale: WO9412122

(56) Documents cités:
- EP-A- 0 176 711
- EP-A- 0 363 151
- WO-A-90/09154
- GB-A- 2 054 383

## Description

La présente invention concerne une tige fémorale de prothèse totale de hanche non cimentée, destinée à être implantée sans contact ni repousse osseuse distale à l'intérieur d'un fût fémoral.

L'invention trouve application dans le domaine de l'orthopédie.

On connaît de l'état de la technique des tiges fémorales présentant des dispositifs de positionnement assurant notamment les fonctions de guidage et de centrage à l'intérieur du fût fémorale ; ces dispositifs étant constitués par des cales métalliques disposées à l'extrémité distale desdites tiges. Ces cales permettent de placer et de maintenir les prothèses dans une position définie et reproductible par rapport au fémur.

Elles ont également un rôle de guidage lors de l'introduction de la prothèse à l'intérieur du fût fémoral.

Ces dispositifs de positionnement connus présentent toutefois l'inconvénient que les cales métalliques utilisées sont en permanence en contact avec l'os, ce qui, en cas d'enfoncement de la tige dans le fémur, comme cela se produit pour les prothèses de hanche sans ciment, peut provoquer un blocage de la cale et être à l'origine de l'effet connu sous le terme anglo-saxon de "stress shielding" particulièrement préjudiciable à la longévité de l'implant fémoral. Cet effet se traduit par une mise en court-circuit par la partie distale des tiges des contraintes appliquées aux prothèses, avec pour conséquence d'entraîner une résorption de l'os ainsi non sollicité au niveau du contact avec la partie proximale de la prothèse. Il en résulte un risque de descellement de la tige fémorale.

De plus, on peut observer que les cales de centrage métalliques connues peuvent, dans le temps, s'avérer à l'origine de particules d'usure liées au frottement entre la cale et la tige dans le cas d'une connexion défectueuse.

La tige fémorale de prothèse de hanche non cimentée décrite dans le document WO-A-90 09154 tente de remédier à ces difficultés. Toute la longueur de la tige destinée à être implantée à l'intérieur d'un fût fémoral bénéficie d'un revêtement ostéoconducteur permettant d'assurer un contact et une repousse osseuse aussi bien en partie proximale qu'en partie distale. Cette dernière opération s'effectue en deux temps, c'est-à-dire d'abord une repousse en partie proximale puis en partie distale. A cet effet ladite partie distale est pourvue d'une couche d'un matériau résorbable non-ostéoconducteur, de sorte qu'après implantation de la tige, la repousse osseuse ne s'effectue pas sur la partie distale tant que la couche de matériau n'est pas résorbée, mais seulement sur la partie proximale. Puis, après résorption dudit matériau, la repousse osseuse peut s'effectuer en partie distale.

On comprend alors que dans cette prothèse connue, le revêtement résorbable non-ostéoconducteur joue essentiellement le rôle d'inhibiteur et de retardateur de la repousse osseuse en partie distale.

A terme, la tige sera cependant au contact de l'os sur toute sa longueur, y compris la partie distale, ce qui pourra à nouveau déclencher l'effet de "stress shielding" si préjudiciable à la tenue de l'implant.

Aussi, le problème technique à résoudre par l'objet de la présente invention est de proposer une tige fémorale de prothèse totale de hanche non cimentée, destinée à être implantée sans contact ni repousse osseuse distale à l'intérieur d'un fût fémoral, la tige fémorale 10 comprenant une partie proximale 12 de contact pourvue de moyens présentant des propriétés ostéoconductrices ainsi qu'un dispositif de positionnement assurant notamment son guidage et son centrage à l'intérieur dudit fût fémoral, tige fémorale dont la structure et le dispositif de positionnement permettrait d'obtenir un guidage et un centrage parfaits, sans provoquer à cours ou long terme d'effet de "stress shielding" ou de création de particules métalliques.

La solution au problème technique posé consiste, selon la présente invention, en ce que ladite tige fémorale comprend une partie distale non-ostéoconductrice et est destinée à être implantée sans contact ni repousse osseuse distale à l'intérieur d'un fût fémoral.

Ainsi, lors de la pose de la prothèse, la tige est parfaitement guidée sur tout son trajet à travers le fémur en évitant en particulier le phénomène de variation, ou mise en bascule de la prothèse. L'effet de centrage du bouchon sera sensible à l'intérieur du fût fémoral pendant une durée d'un mois à deux ans, variable selon le type de matériau résorbable utilisé. Durant cette période, la fixation d'une prothèse de hanche sans ciment par ostéoconduction en partie proximale de la tige pourra s'établir et se pérenniser.

Après résorption du bouchon, aucun effet de "stress-shielding" n'est susceptible de se produire, même en cas de subsidence ou autre déplacement de la prothèse, puisqu'aucun contact ne peut en principe exister entre l'extrémité de la tige et l'os, avec l'avantage supplémentaire que, du fait d'un centrage idéal, la tige ne peut venir toucher l'os, et donc provoquer de douleurs chez le patient. De même, toute repousse osseuse éventuelle qui résulterait d'un contact accidentel avec l'os est empêchée par le caractère non ostéoconducteur de la partie distale de la tige.

De même, le bouchon n'étant pas réalisé dans un matériau métallique, il ne peut pas se créer de particules d'usure résultant du frottement entre le bouchon et la tige fémorale.

La description qui va suivre en regard des dessins annexés, donnés à titre d'exemples non limitatifs, fera bien comprendre en quoi consiste l'invention et comment elle peut être réalisée.

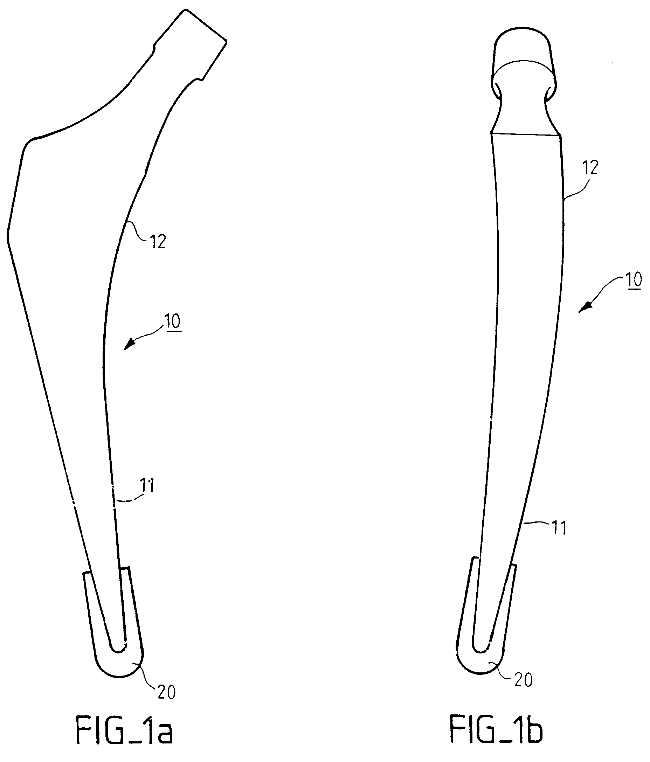

Le figures 1a et 1b sont respectivement des vues de côté et de face d'une tige fémorale munie d'un dispositif de positionnement conforme à l'invention.

La figure 2 est une vue de côté d'un dispositif de positionnement de l'invention comportant un système de verrouillage à baïonnette.

La figure 3 est une vue de côté d'une tige fémorale sans ciment prévue pour recevoir le dispositif de positionnement de la figure 2.

Les figures 1a et 1b montrent, en vues de côté et de face, une tige fémorale 10 pour une prothèse totale de hanche. Comme on peut le voir sur les figures précitées, ladite tige 10 est munie d'un dispositif de positionnement comprenant un bouchon 20 en matériau résorbable non ostéoconducteur s'adaptant à l'extrémité de la partie distale 11 de la tige fémorale 10, ladite partie distale étant dépourvue de propriétés ostéoconductrices.

Dans le mode de réalisation des figures 1a et 1b, le bouchon 20 résorbable a une forme cylindro-conique, mais il pourrait avoir tout aussi bien une forme sphéroïdale , cylindrique ou conique.

Le matériau utilisé pour réaliser le bouchon 20 est un matériau résorbable biocompatible qui peut être, par exemple, un copolymère d'acide lactique et d'acide glycolique en proportion variable selon le temps de résorption désiré. Ce type de matériau, qui a également la propriété de ne pas être ostéoconducteur afin d'éviter la repousse osseuse en partie distale, est disponible commercialement sous le nom de "Resomer" (marque déposée) fabriquée par la société Boehringer Ingelheim ou de "Medisorb" ( marque déposée) fabriqué par la société Médisorb, filiale de Dupont de Nemours.

Les bouchons de positionnement conformes à l'invention peuvent être usinés, moulés ou mis en oeuvre par toute autre technique.

Les figures 1a et 1b illustrent un mode d'exécution de la tige fémorale 10 selon l'invention dans lequel le bouchon 20 résorbable s'adapte à l'extrémité de la partie distale 11 de la tige fémorale par emboîtement du type cône morse.

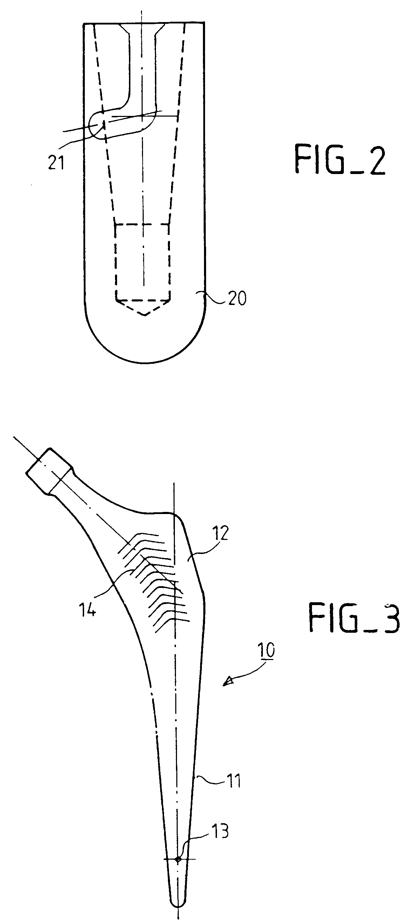

Conformément à la variante de réalisation représentée aux figures 2 et 3, le bouchon 20 de positionnement s'adapte à l'extrémité de la partie distale 11 de la tige fémorale 10 à l'aide d'un système 21 à baïonnette, la tige fémorale portant alors un plot 13 de verrouillage.

La tige 10 montrée sur la figure 3 est du type non cimentée pour laquelle la fixation au fémur est réalisée par repousse osseuse dans la partie proximale 12 de ladite tige. Afin de favoriser ce phénomène d'ancrage osseux, la tige fémorale 10 est pourvue, dans cette zone proximale 12 de contact, de moyens présentant des propriétés d'ostéoconduction qui peuvent être des reliefs latéraux, comme dans l'exemple de la figure 3, ou encore un revêtement d'hydroxyapatite ou d'un matériau équivalent.

## Revendications

1. Tige fémorale (10) de prothèse totale de hanche non cimentée, la tige fémorale (10) comprenant une partie proximale (12) de contact pourvue de moyens présentant des propriétés ostéoconductrices ainsi qu'un dispositif de positionnement assurant notamment son guidage et son centrage à l'intérieur dudit fût fémoral, ledit dispositif de positionnement comprenant un bouchon (20) de centrage en matériau résorbable et non ostéoconducteur s'adaptant à l'extrémité de la partie distale (11) de la tige fémorale (10), caractérisée en ce que ladite tige fémorale (10) comprend en outre une partie distale (11) non ostéoconductrice et est destinée à être implantée sans contact ni repousse osseuse distale à l'intérieur d'un fût fémoral.

2. Tige fémorale (10) selon la revendication 1, caractérisée en ce que ledit bouchon (20) a une forme cylindro-conique.

3. Tige fémorale (10) selon la revendication 1, caractérisée en ce que ledit bouchon (20) a une forme sphéroïdale.

4. Tige fémorale (10) selon l'une quelconque des revendications 1 à 3, caractérisée en ce que ledit bouchon (20) s'adapte à l'extrémité de la partie distale (11) de la tige fémorale (10) à l'aide d'un système (21) de verrouillage à baïonnette, ladite tige fémorale (10) portant un plot (13) de verrouillage.

5. Tige fémorale (10) selon l'une quelconque des revendications 1 à 3, caractérisée en ce que ledit bouchon (20) s'adapte à l'extrémité de la partie distale (11) de la tige fémorale (10) par emboîtement du type cône morse.

## Claims

1. A femoral pin (10) for a full, non-cemented hip prosthesis, the femoral pin (10) consisting of a proximal contact part (12) provided with means having osteo-conductive properties as well as a positioning device so that it can be correctly guided and centred inside a femoral brace, the said positioning device being a centring cap (20) made from a material that is capable of resorption and is not osteo-conductive and is adapted to the end of the distal part (11) of the femoral pin (10), characterised in that the said femoral pin (10) also has a distal part (11) which is not osteo-conductive and is designed to be implanted in the said femoral brace without either contact or bone regrowth.

2. A femoral pin (10) as claimed in claim 1, characterised in that the said cap (20) is of a rounded conical shape.

3. A femoral pin as claimed in claim 1, characterised in that the said cap (20) is spheroidal in shape.

4. A femoral pin (10) as claimed in one of claims 1 to 3, characterised in that the said cap (20) is adapted to the end of the distal part (11) of the femoral pin (10) by means of a bayonet fitting system (21), the said femoral pin (10) having a locking socket (13).

5. A femoral pin as claimed in any one of claims 1 to 3, characterised in that the said cap (20) is adapted to the end of the distal part (11) of the femoral pin (10) by a fitting of the Morse taper type.

## Patentansprüche

1. Oberschenkelstiel (10) einer nicht-einzementierten Hüft-Totalprothese, wobei der Oberschenkelstiel (10) einen proximalen Berührungsteil (12) aufweist, der mit Mitteln versehen ist, die das Knochenwachstum lenkende Eigenschaften aufweisen, sowie mit einer Positionierungsvorrichtung, die insbesondere seine Führung und Zentrierung im Inneren des Oberschenkelknochen-Schaftes sicherstellt, und wobei die genannte Positionierungsvorrichtung einen Zentrierstopfen (20) aus einem resorbierbaren und das Knochenwachstum nicht-lenkenden Material aufweist, der sich an das Ende des distalen Abschnitts (11) des Oberschenkelstiels (10) anpaßt, dadurch gekennzeichnet, daß der genannte Oberschenkelstiel (10) ausserdem einen distalen, das Knochenwachstum nicht-lenkenden Abschnitt (11) aufweist und dazu bestimmt ist, ohne Berührung und ohne distales Anwachsen des Knochens in das Innere eines Oberschenkelknochen-Schaftes implantiert zu werden.

2. Oberschenkelstiel (10) nach Anspruch 1, dadurch gekennzeichnet, daß der genannte Stopfen (20) eine zylindrisch-konische Form hat.

3. Oberschenkelstiel (10) nach Anspruch 1, dadurch gekennzeichnet, daß der genannte Stopfen (20) eine kugelige Form hat.

4. Oberschenkelstiel (10) nach irgendeinem der Ansprüche 1 bis 3, dadurch gekennzeichnet, daß der genannte Stopfen (20) sich an das Ende des distalen Abschnitts (11) des Oberschenkelstiels (10) mit Hilfe eines Bajonett-Verriegelungssystems (21) anpaßt, wobei der genannte Oberschenkelstiel (10) einen Verriegelungszapfen (13) trägt.

5. Oberschenkelstiel (10) nach irgendeinem der Ansprüche 1 bis 3, dadurch gekennzeichnet, daß der genannte Stopfen (20) sich an das Ende des distalen Abschnitts (11) des Oberschenkelstiels (10) durch einen Morsekonus-Sitz anpaßt.
